# EUROPEAN PATENT APPLICATION

(11) **EP 4 202 867 A1**
(43) Date of publication of application: **28.06.2023**
(21) Application number: 21217256.3
(22) Date of filing: 23.12.2021
(51) Int. Cl.: G06V 30/41, G06F 40/20, G06V 10/26, G06V 10/22, G06F 16/30, G06F 16/20, G06F 16/50, G16H 10/00, G16H 15/00, G16H 30/00

(54) **METHOD, DEVICE AND SYSTEM FOR AUTOMATED PROCESSING OF MEDICAL IMAGES AND MEDICAL REPORTS OF A PATIENT**

(71) Applicant: Siemens Healthcare GmbH, 91052 Erlangen (DE)
(72) Inventor: YEREBAKAN, Halid, Malvern, 19355 (US); SHINAGAWA, Yoshihisa, Downingtown, 19335 (US)
(74) Representative: Horn Kleimann Waitzhofer Patentanwälte PartG mbB

(57) **Abstract**

A method, a device and a system for automated processing of medical images and medical reports of a patient are provided. In one aspect, the method comprises: receiving a plurality of medical images of the patient; receiving a number of medical reports of the patient; extracting a number of text elements from the number of medical reports, each of the text elements having a textual description of a medical problem of the patient and a location indicator indicating a location of a specific medical image in said plurality of medical images; for each of the extracted text elements, creating a structured element including the extracted text element and a location information being provided based on the location indicator of the extracted text element; and for each of the created structured elements, associating the respective created structured element with the respective specific medical image based on the location information of the respective created structured element.

## Description

The present invention relates to a computer-implemented method, to a computer-implemented device and to a system for automated processing of medical images and medical reports of a patient.

For example, in radiologist studies, the historical context of a patient plays an important role in understanding current clinical conditions of the patient. This is the reason why doctors look at previous radiologic reports and navigate to corresponding findings in medical images. However, every finding in the report requires extra efforts to find the corresponding location of the medical image manually. This is a time-consuming process, particularly because it has to be repeated for all subsequent studies of the patient.

To eliminate unnecessary scrolling, some conventional tools allow saving key images in structured repositories to get an immediate overview in later studies. However, still the coverage of this workflow may be limited due to user preferences and third-party data resources. On the other hand, there are large collections of report-image pairs without structured mapping.

Therefore, the availability of text-image data sets may be helpful. Here, one major research direction is generating reports from medical images automatically. For example, reference [1] proposes using neural networks to generate captions for given X-ray images. In this regard, further conventional solutions are described in references [2]-[6].

The object of the invention is therefore to provide a method, device and system that enables an improved automated processing of medical images and medical reports of a patient.

According to a first aspect, a computer-implemented method for automated processing of medical images and medical reports of a patient is proposed. The method comprises:
a) receiving a plurality of medical images of the patient,
b) receiving a number of medical reports of the patient,
c) extracting a number of text elements, in particular a plurality of text elements, from the number of medical reports, each of the text elements having a textual description of a medical problem of the patient and a location indicator indicating a location of a specific medical image in said plurality of medical images,
d) for each of the extracted text elements, creating a structured element including the extracted text element and a location information being provided based on the location indicator of the extracted text element, and
e) for each of the created structured elements, associating the respective created structured element with the respective specific medical image based on the location information of the respective created structured element.

The medical images may be captured by and received from a medical imaging unit. The medical imaging unit may include, for example, but not limited to, magnetic resonance imaging device, computed tomography device, X-ray imaging device, ultrasound imaging device, etc. The medical images may be three-dimensional and/or related to a volume. The medical images may include one or more anatomical objects associated with a patient. The anatomical objects may be in particular one or more body parts associated with the patient that may have been imaged. The anatomical objects may include, but not be limited to, one or more imaged organs, such as the lung. The medical images may, for example, be in the form of an array of pixels or voxels. Such arrays of pixels or voxels may be representative of intensity, absorption or other parameter as a function of three-dimensional position, and may, for example, be obtained by suitable processing of measurement signals obtained by one or more of the above-mentioned medical imaging units. In particular, the medical image may include information associated with a region of interest for tumor detection. Alternatively, the medical images may be received from a medical database including a plurality of medical images.

In particular, the medical report is an unstructured medical report or unstructured document. Moreover, the specific medical image shows the medical problem described in the textual description being part of the respective extracted text element.

The present method is adapted to correlate medical reports with image locations, presently provided by the location information. As a result, report parsing for patient history navigation can be provided. By means of steps a) to d), the method creates a desired structure, particularly based on the extracted text elements, in particular using information extraction. Then, by means of step e), the extracted information, i.e. the created structured elements, is automatically associated with image locations of the medical images. After the association, a user may click to findings and navigate to corresponding images, in particular using a graphical user interface (discussed in detail below). In particular, a number of means at least one, and a plurality means at least two.

Advantageously, the present method does not require prior annotation of text-image pairs to navigate to the medical images. In particular, the present associations may be extracted by natural language processing automatically. Moreover, organ segmentation, computer-aided detections or landmark locations may be used to match sentences as well. Thus, the present method may be applied to a broad range of image-text pairs advantageously.

According to an embodiment, in step c), the text elements are extracted from the number of medical reports using a trained machine learning algorithm, generalized context free grammars and/or keyword patterns.

In particular, in step c), series and slice numbers of the medical images may be extracted using generalized context-free grammars from the unstructured medical report. The specification for extractions may include both key word patterns and machine learning models. Particularly, machine learning models may normalize text data into a form where parsing is easier. These models are more generic since they may work on multiple words and languages at the same time. Moreover, subword level tokenization may be used by the present method. With the help of subword level tokenization, it may generalize to words previously unseen. Later, these extractions may be listed in a table to simplify easy selection of findings.

As mentioned above, the association of text elements to medical images may be beyond using image numbers. The present method is capable of extracting organ information, location-laterality information and negations, e.g. "a 10 mm spiculated nodule in the right lower lobe of the lung". Thus, even with the lack of an image number information, the present method may navigate to specific organs. In order to identify the coordinates of the organs in the medical images, segmentation or landmarking may be used. Moreover, written locations of lesions and the lesions detected by a lung CAD (computer-assisted detection) from medical images may be matched.

According to a further embodiment, the method further comprises:
outputting, by a graphical user interface, a graphical representation including:
a certain medical report of the number of received medical reports,
a plurality of created structured elements, each of the plurality of created structured elements including a text element being extracted from said certain medical report and a location information being provided based on the location indicator of the extracted text element, and
one of the specific medical images associated to a certain one of the created structured elements.

According to a further embodiment, the method further comprises:
receiving a user input for selecting a certain location information of the structured elements being output by the graphical representation,
changing the graphical representation such that the medical image being associated to the certain location information selected by the user input becomes part of the graphical representation.

According to a further embodiment, the medical image being output by the graphical representation at a instance of time when receiving the user input for selecting the certain location information of the structured elements is substituted in the graphical representation by the medical image being associated to the certain location information selected by the user input.

According to a further embodiment, the method comprises:
receiving the plurality of medical images of the patient and storing the received medical images in a medical database, and
extracting the plurality of text elements from the number of medical reports, each of the text elements having the textual description of the medical problem of the patient and the location indicator indicating a location of the specific medical image in the medical database.

According to a further embodiment, the graphical representation includes a table for representing the created structured elements to the user. The table may include a respective line for each of the created structured elements and at least two columns, a first column for representing the extracted text elements to the user and at least one further column for representing the location information allocated to the extracted text elements to the user.

According to a further embodiment, the medical image is a slice, in particular a 2D slice being extracted at a certain plane of an anatomical object of the patient.

According to a further embodiment, each of the medical images is a 3D medical image of the anatomical object of the patient.

According to a further embodiment, the medical images are acquired by a medical imaging unit, in particular by a MR scanner or a CT scanner.

According to a further embodiment, the location information for a specific slice includes a series number indicating to which specific series the specific slice belongs to and a slice number identifying the specific slice in the specific series.

According to a further embodiment, step e) includes associating the created structured element with the specific slice using the series number and the slice number.

According to a further embodiment, step e) includes associating the created structured element with the specific medical image using segmentation, wherein segmentation is particularly used for identifying a region of the medical problem in the specific medical image.

According to a further embodiment, step e) includes associating the created structured element with the specific medical image using a landmarking tool.

According to a further embodiment, a written location of a lesion from the extracted text element is matched with a corresponding lesion in the specific medical image using computer assisted detection.

According to a second aspect, a computer-implemented device for automated processing of medical images and medical reports of a patient is proposed. The computer-implemented device comprises:
one or more processing units,
a receiving unit which is configured to receive one or more medical images captured by a medical imaging unit and medical reports of a patient, and
a memory coupled to the one or more processing units, the memory comprising a module configured to perform the method steps of the first aspect or of any embodiment of the first aspect using a trained machine learning network.

The respective unit, e.g., the processing unit or the receiving unit, may be implemented in hardware and/or in software. If said unit is implemented in hardware, it may be embodied as a device, e.g., as a computer or as a processor or as a part of a system, e.g., a computer system. If said unit is implemented in software, it may be embodied as a computer program product, as a function, as a routine, as a program code or as an executable object.

The embodiments and features according to the first aspect are also embodiments of the second aspect.

According to a third aspect, a system for automated processing of medical images and medical reports of a patient is proposed. The system comprises:
one or more servers,
a medical imaging unit coupled to the one or more servers,
the one or more servers comprising instructions, which when executed causes the one or more servers to perform the method steps of the first aspect or of any embodiment of the first aspect.

The embodiments and features according to the first aspect are also embodiments of the third aspect.

According to a fourth aspect, a computer program product is proposed, the computer program product comprising machine readable instructions, that when executed by one or more processing units, cause the one or more processing units to perform the method of the first aspect or of any embodiment of the first aspect.

The embodiments and features according to the first aspect are also embodiments of the fourth aspect.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a fifth aspect, a computer readable medium is proposed on which program code sections of a computer program are saved, the program code sections being loadable into and/or executable in a system to make the system execute the method of the first aspect or of any embodiment of the first aspect when the program code sections are executed in the system.

The embodiments and features according to the first aspect are also embodiments of the fifth aspect.

The realization by a computer program product and/or a computer-readable medium has the advantage that already existing management systems can be easily adopted by software updates in order to work as proposed by the invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features, and advantages of the present invention will become apparent from the subsequent description and dependent claims, taken in conjunction with the accompanying drawings, in which:
- FIG. 1: illustrates a block diagram of a client-server architecture embodying a system for automated processing of medical images and medical reports of a patient according to an embodiment of the present invention,
- FIG. 2: illustrates a block diagram of a data processing system embodying a computer-implemented device for automated processing of medical images and medical reports of a patient,
- FIG. 3: illustrates a flowchart of a first embodiment of a method for automated processing of medical images and medical reports of a patient,
- FIG. 4: illustrates a flowchart of a second embodiment of a method for automated processing of medical images and medical reports of a patient,
- FIG. 5: illustrates an example of a graphical representation according to an embodiment of the present invention, and
- FIG. 6: illustrates the example of the graphical representation of FIG. 5 with a different represented medical image.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

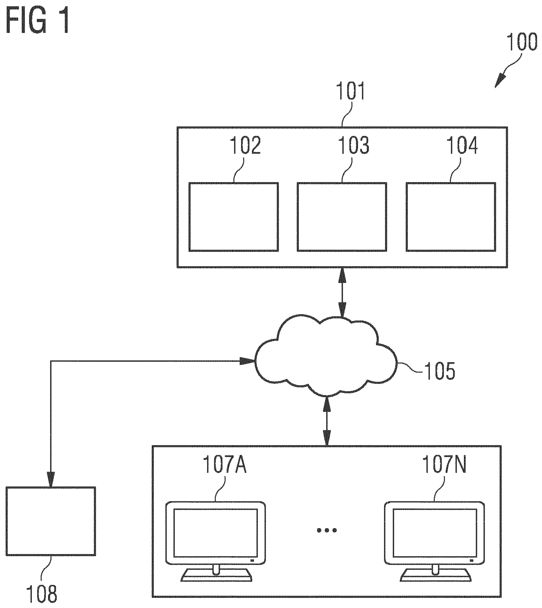

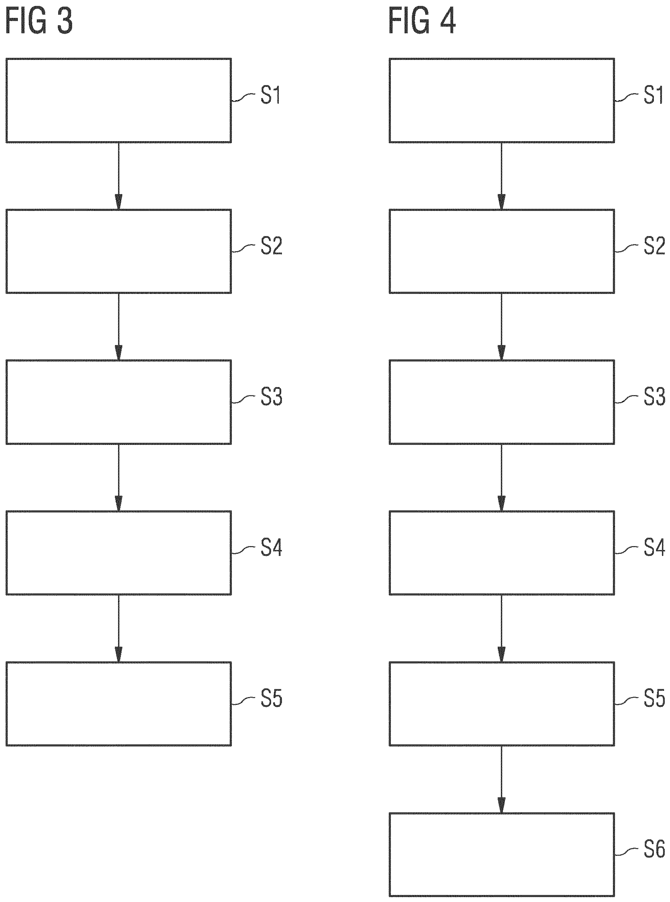

FIG. 1 provides an illustration of a block diagram of a client-server architecture embodying a system for automated processing of medical images I1, I2 and medical reports R of a patient. The client-server architecture 100 comprises a server 101 and a plurality of client devices 107A-N. Each of the client devices 107A-N is connected to the server 101 via a network 105, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 105, for example, the internet. The cloud computing environment provides on-demand network access to a shared pool of the configurable computing physical and logical resources. The server 101 may include a medical database 102 that comprises medical images I1, I2 and associated medical reports R related to a plurality of patients that is maintained by a healthcare service provider. In an embodiment, the medical database 102 comprises medical images I1, I2 captured by a MR scanner and/or by a CT scanner. The server 101 may include a module 103 that is configured to perform steps for automated processing of medical images I1, I2 and medical reports R of a patient (see FIG. 3 and FIG. 4). The server 101 may include a network interface 104 for communicating with the client device 107A-N via the network 105.

The client devices 107A-N are user devices, used by users, for example, medical personnel such as a radiologist, pathologist, physician, etc. In an embodiment, the user device 107A-N may be used by the user to study medical reports R and medical images I1, I2 associated with the patient. The data can be accessed by the user via a graphical user interface of an end user web application on the user device 107A-N. In another embodiment, a request may be sent to the server 101 to access the medical reports R and the medical images I1, I2 associated with the patient via the network 105. An imaging unit 108 may be connected to the server 101 through the network 105. The unit 108 may be a medical imaging unit 108 capable of acquiring a plurality of medical images 1, I2. The medical imaging unit 108 may be, for example, a scanner unit such as a magnetic resonance imaging unit, computed tomography imaging unit, an X-ray fluoroscopy imaging unit, an ultrasound imaging unit, etc.

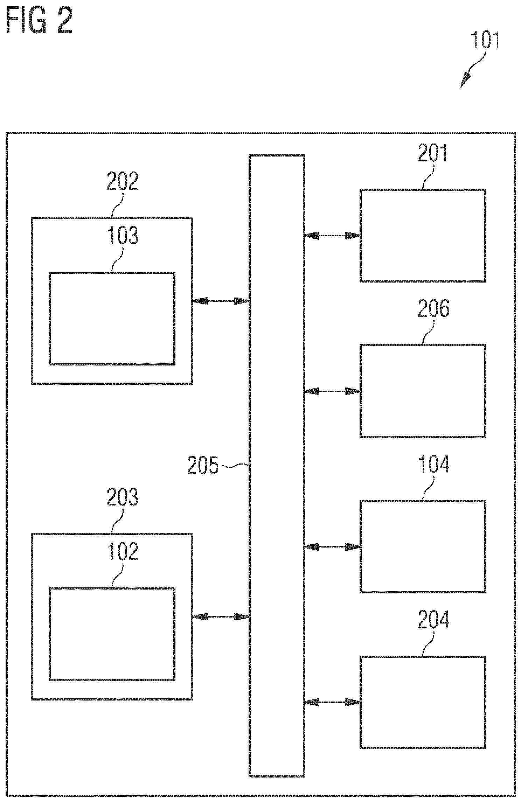

FIG. 2 is a block diagram of a data processing system 101 in which an embodiment can be implemented, for example, as a system 101 for automated processing of medical images I1, I2 and medical reports R of a patient, configured to perform the processes as described therein. It is appreciated that the server 101 is an exemplary implementation of the system in FIG. 2. In FIG. 2, said data processing system 101 comprises a processing unit 201, a memory 202, a storage unit 203, an input unit 204, an output unit 206, a bus 205, and a network interface 104.

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

The memory 202 may be volatile memory and non-volatile memory. The memory 202 may be coupled for communication with said processing unit 201. The processing unit 201 may execute instructions and/or code stored in the memory 202. A variety of computer-readable storage media may be stored in and accessed from said memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 comprises a module 103 stored in the form of machine-readable instructions on any of said above-mentioned storage media and may be in communication to and executed by processing unit 201. When executed by the processing unit 201, the module 103 causes the processing unit 201 to automatically process medical images I1, I2 and medical reports R of a patient. Method steps executed by the processing unit 201 to achieve the abovementioned functionality are elaborated upon in detail in FIG. 3, and in FIG. 4.

The storage unit 203 may be a non-transitory storage medium which stores a medical database 102. The medical database 102 is a repository of medical images I1, I2and associated medical reports R related to one or more patients that is maintained by a healthcare service provider. The input unit 204 may include input means such as keypad, touch-sensitive display, camera (such as a camera receiving gesture-based inputs), etc. capable of receiving input signal such as a medical image 1, I2. The bus 205 acts as interconnect between the processor 201, the memory 202, the storage unit 203, the input unit 204, the output unit 206 and the network interface 104.

Those of ordinary skilled in the art will appreciate that said hardware depicted in FIG. 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 101 in accordance with an embodiment of the present disclosure may comprise an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described. Disclosed embodiments provide systems and methods for processing medical images.

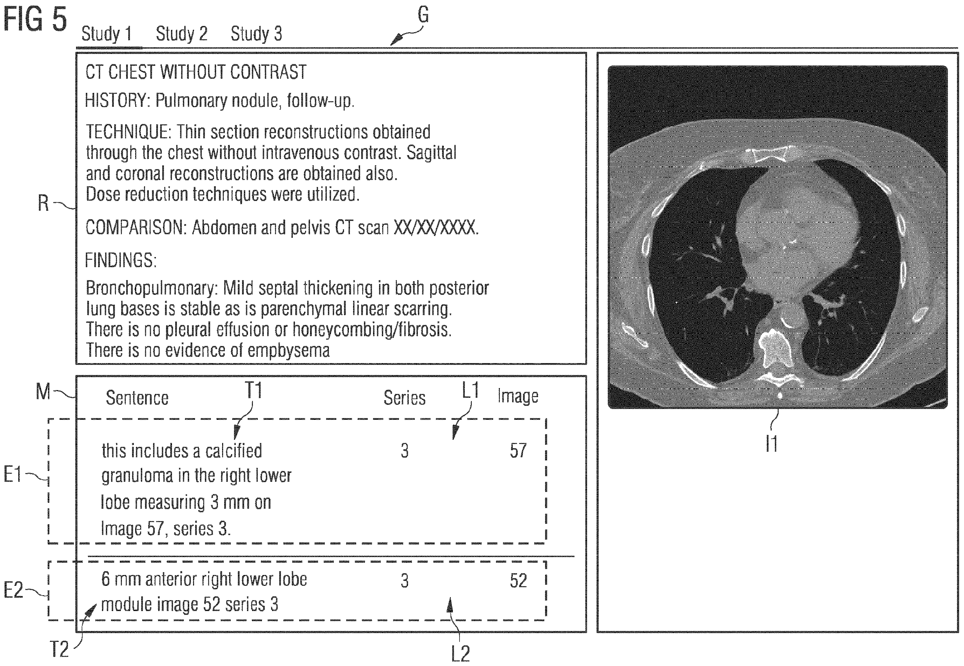

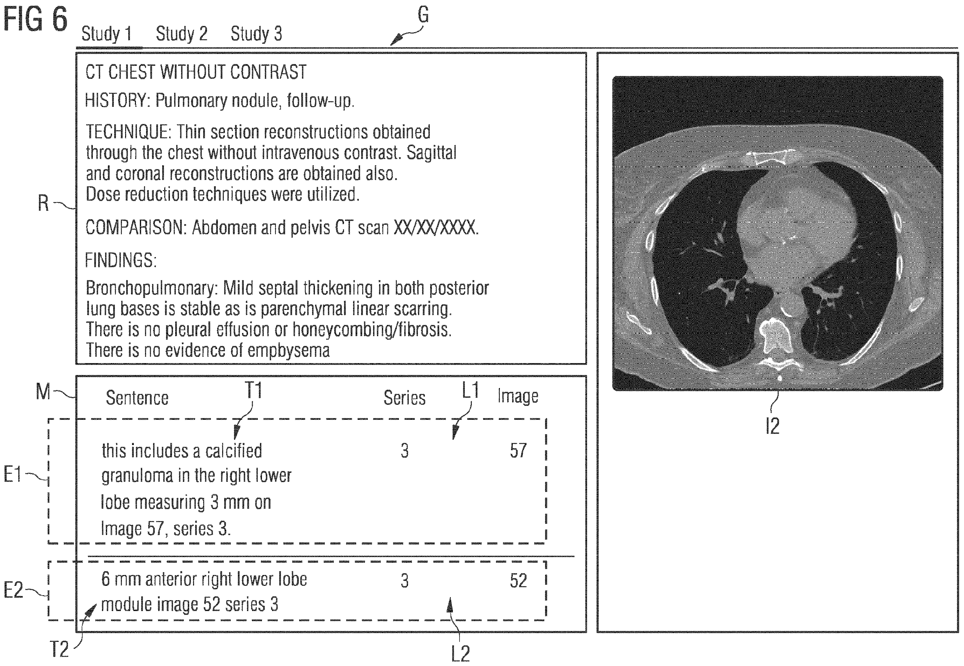

FIG. 3 and FIG. 4 illustrate flowcharts of two embodiments of a method for automated processing of medical images and medical reports of a patient. The embodiment of FIG. 3 includes the method steps S1 to S5, and the embodiment of FIG. 4 includes method step S6 additionally. These two embodiments of a method according to FIG. 3 and FIG. 4 are discussed in the following with reference to FIG. 5 and FIG. 6 illustrating an example of a graphical representation G according to an embodiment of the present invention.

In particular, the method of FIG. 3 and the method of FIG. 4 may be executed by above discussed module 103. The embodiment of FIG. 3 includes the method steps S1 to S5:
In step S1, a plurality of medical images I1, I2 of the patient are received (see FIG. 5 and FIG. 6). In particular, the respective medical image I1, I2 is a slice. For example, the slice is a 2D slice being extracted at a certain plane of an anatomical object, e.g. the lung, of the patient.

In step S2, a number of medical reports R of the patient are received (see FIG. 5 and FIG. 6). The respective medical report R may include a textual description of a medical problem of the patient and at least one location indicator indicating a location of a specific medical image I1, I2in said received medical images showing said medical problem.

In step S3, a plurality of text elements T1, T2 are extracted from the number of medical reports R (see FIG. 5 and FIG. 6). Each of the text elements T1, T2 includes a textual description of the medical problem of the patient and a location indicator indicating a location of a specific medical image I1, I2 in said plurality of medical images I1, I2. In particular, the plurality of medical images are received and then stored in a medical database 102. In such a case, the plurality of text elements T1, T2 may be extracted from the number of medical reports R, wherein each of the text elements T1, T2 having the textual description of the medical problem of the patient and the location indicator indicating a location of the specific medical image I1, I2 in the medical database 102.

In particular, in step S3, the text elements T1, T2 are extracted from the number of medical reports R using a trained machine learning algorithm, generalized context-free grammar or keyword patterns.

In step S4, for each of the extracted text elements T1, T2, a structured element E1, E2 including the extracted text element T1, T2 and the location information L1, L2 being provided based on the location indicator of the extracted text element T1, T2 is created (see FIG. 5). The location information L1, L2 may correspond to the location indicator of the extracted text element T1, T2 or may be generated in dependence on said location indicator.

In step S5, for each of the created structured elements E1, E2, the respective created structured element E1, E2 is associated with (or allocated to) the respective specific medical image I1, I2 based on the location information L1, L2 of the respective created structured element E1, E2.

Furthermore, step S5 may include associating the created structured element E1, E2 with the specific slice I1, I2 using the series number and the slice number.

Alternatively or additionally, said step S5 may include associating the created structured element E1, E2 with the specific medical image I1, I2 using segmentation. Segmentation is particularly used for identifying a region of the medical problem in the specific medical image I1, I2.

Alternatively or additionally, step S5 may include associating the created structured element E1, E2 with the specific medical image I1, I2 using landmarking. For example, a written location of a lesion from the extracted text element T1, T2 may be matched with a corresponding lesion in the specific medical image I1, I2 using computer-assisted detection.

As mentioned above, FIG. 4 additionally includes method step S6. In step S6, a graphical representation G (see FIG. 5 and FIG. 6) is output to a user by a graphical user interface (GUI). The graphical representation G particularly includes: a certain medical report R, a plurality of created structured elements E1, E2, wherein each of the plurality of created structured elements E1, E2 includes a text element T1, T2 being extracted from said certain medical report R and a location information L1, L2 being provided based on the location indicator of the extracted text element T1, T2, and a specific medical image I1 associated to a certain one of the created structured elements E1, E2. In FIG. 5, the medical image I1 is represented to the user, said medical image I1 being associated to the created structured element E1.

In FIG. 5, the left part shows the medical report R and, underneath the medical report R, a structured data model M including two structured elements E1, E2. Without loss of generality, the structured data model M shown in FIG. 5 has two structured elements E1, E2.

Each of the structured elements E1, E2 has a text element T1, T2 and a location information L1, L2 for a respective medical image I1, I2 . As one can see from FIG. 5, the graphical representation R includes a table representing the structured data model M. The table for the structured data model M includes a respective line for each of the created structured elements E1, E2 and three columns, a first column for representing the extracted text element T1, T2 to a user, a second column and a third column for representing the location information L1, L2 allocated to the respective one of the extracted text elements T1, T2.

In the example of FIG. 5, as one can see in the right part of FIG. 5, the medical image I1 is a slice, in particular a 2D slice being extracted at a certain plane of an anatomical object of the patient. In this regard, in the example of FIG. 5, the respective location information L1, L2 for a specific slice includes a series number indicating to which specific series the specific slice belongs and a slice number identifying the specific slice in the specific series. As a result, the table of FIG. 5 showing the structured data model M has two columns for the location information L1, L2, i.e. one column for the series number, called Series in FIG. 5. and one column for the slice number, called Image in FIG. 5.

Moreover, the graphical representation G of FIG. 5 may be embodied as an interface for the user which is configured to react on user inputs, e.g. click events. For example, if a user input for selecting a certain location information, e. g. L2 of the structured element E2 being output by the graphical representation G, is received, the graphical representation G is changed such that the medical image I2 being associated to the certain location information L2 selected by the user input becomes part of the graphical representation G. This change is shown by FIG. 6. In other words, if the graphical representation G of FIG. 5 shows, in its right part, a medical image I1 associated with the first structured element E1 and the user clicks on the location information L2 being part of the second structured element E2, then the graphical representation G of FIG. 5 is changed into the graphical representation G of FIG. 6 such that the medical image I1 associated with the first structured element E1 and being output to the user before he provides his user input is changed to the medical image I2 being associated with the location information L2, and being selected by the user input. Thus, the medical image I1 being output by the graphical representation G at an instance of time (see FIG. 5) when receiving the user input for selecting a certain location information L2 of the structured element E2 is substituted in the graphical representation G by said medical image I2 (see FIG. 6) being associated to the certain location information L2 selected by the user input.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### REFRENCE SIGNS

- 100: system
- 101: computer-implemented device
- 102: medical database
- 103: module
- 104: network interface
- 105: network
- 107A - 107N: client device
- 108: medical imaging unit
- 201: processing unit
- 202: memory
- 203: storage unit
- 204: input unit
- E1: structured element
- E2: structured element
- G: graphical representation
- I1: medical image
- I2: medical image
- L1: location information
- L2: location information
- M: structured data model
- R: medical report
- S1 - S6: method steps
- T1: text element
- T2: text element

### REFERENCES

[1] Jing, Baoyu, Pengtao Xie, and Eric Xing. "On the automatic generation of medical imaging reports." arXiv preprint arXiv: 1711.08195 (2017).
[2] Lu, Jiasen, et al. "Vilbert: Pretraining task-agnostic visiolinguistic representations for vision-and-language tasks." arXiv preprint arXiv:1908.02265 (2019).
[3] https://aws.amazon.com/comprehend/medical/
[4] https://openaccess.thecvf.com/content_ECCV_2018/papers/Ying_Zhang_Deep_Cross-Modal_Projection_ECCV_2018_paper.pdf
[5] WO2007096214A1
[6] EP3757824A1

## Claims

1. A computer-implemented method for automated processing of medical images (I1, I2) and medical reports (R) of a patient, the method comprising:
a) receiving (S1) a plurality of medical images (I1, I2) of the patient,
b) receiving (S2) a number of medical reports (R) of the patient,
c) extracting (S3) a number of text elements (T1, T2), in particular a plurality of text elements (T1, T2), from the number of medical reports (R), each of the text elements (T1, T2) having a textual description of a medical problem of the patient and a location indicator indicating a location of a specific medical image (I1, I2) in said plurality of medical images (I1, I2),
d) for each of the extracted text elements (T1, T2), creating (S4) a structured element (E1, E2) including the extracted text element (T1, T2) and a location information (L1, L2)being provided based on the location indicator of the extracted text element (T1, T2), and
e) for each of the created structured elements (E1, E2), associating (S5) the respective created structured element (E1, E2) with the respective specific medical image (I1, I2) based on the location information (L1, L2) of the respective created structured element (E1, E2).

2. The computer-implemented method of claim 1,
wherein, in step c) (S3), the text elements (T1, T2) are extracted from the number of medical reports (R) using a trained machine learning algorithm, generalized context free grammars or keyword patterns.

3. The computer-implemented method of claim 1 or 2, further comprising:
outputting (S6), by a graphical user interface, a graphical representation (G) including:
a certain medical report (R) of the number of received medical reports (R),
a plurality of created structured elements (E1, E2), each of the plurality of created structured elements (E1, E2) including a text element (T1, T2) being extracted from said certain medical report (R) and a location information (L1, L2) being provided based on the location indicator of the extracted text element (T1, T2), and
one of the specific medical images (I1, I2) associated to a certain one of the created structured elements (E1, E2).

4. The computer-implemented method of claim 3, further comprising:
receiving a user input for selecting a certain location (L2) information of the structured elements (E1, E2) being output by the graphical representation (G),
changing the graphical representation (G) such that the medical image (I2) being associated to the certain location information (L2) selected by the user input becomes part of the graphical representation (G).

5. The computer-implemented method of claim 4,
wherein the medical image (I1) being output by the graphical representation (G) at a instance of time when receiving the user input for selecting the certain location information (L2) of the structured elements (E1, E2) is substituted in the graphical representation (G) by the medical image (I2) being associated to the certain location information (L2) selected by the user input.

6. The computer-implemented method of one of claims 1 to 5, comprising:
receiving (S2) the plurality of medical images (I1, I2) of the patient and storing the received medical images (I1, I2) in a medical database (102), and
extracting (S3) the plurality of text elements (T1, T2) from the number of medical reports (R), each of the text elements (T1, T2) having the textual description of the medical problem of the patient and the location indicator indicating a location of the specific medical image (I1, I2) in the medical database (102).

7. The computer-implemented method of one of claims 1 to 6,
wherein the graphical representation (G) includes a table for representing the created structured elements (E1, E2) to the user,
wherein the table includes a respective line for each of the created structured elements (E1, E2) and at least two columns, a first column for representing the extracted text elements (T1, T2) to the user and at least one further column for representing the location information (L1, L2) allocated to the extracted text elements (T1, T2) to the user.

8. The computer-implemented method of claim 7,
wherein the medical image (I1, I2) is a slice, in particular a 2D slice being extracted at a certain plane of an anatomical object of the patient.

9. The computer-implemented method of claim 8,
wherein the location information (L1, L2) for a specific slice includes a series number indicating to which specific series the specific slice belongs to and a slice number identifying the specific slice in the specific series.

10. The computer-implemented method of claim 9,
wherein step e) (S5) includes associating the created structured element (E1, E2) with the specific slice using the series number and the slice number.

11. The computer-implemented method of one of claims 1 to 8,
wherein step e) (S5) includes associating the created structured element (E1, E2) with the specific medical image (I1, I2) using segmentation, wherein segmentation is particularly used for identifying a region of the medical problem in the specific medical image (I1, I2).

12. The computer-implemented method of one of claims 1 to 8,
wherein step e) (S5) includes associating the created structured element (E1, E2) with the specific medical image (I1, I2) using a landmarking tool.

13. The computer-implemented method of one of claims 1 to 12,
wherein a written location of a lesion from the extracted text element (T1, T2) is matched with a corresponding lesion in the specific medical image (I1, I2) using computer assisted detection.

14. A computer-implemented device (101) for automated processing of medical images (I1, I2) and medical reports (R) of a patient, the computer-implemented device (101) comprising:
one or more processing units (201),
a receiving unit (204) which is configured to receive one or more medical images (I1, I2) captured by a medical imaging unit (108) and medical reports (R) of a patient, and
a memory (202) coupled to the one or more processing units (201), the memory (202) comprising a module (103) configured to perform the method steps as claimed in any one of claims 1 to 13.

15. A system (100) for automated processing of medical images (I1, I2) and medical reports (R) of a patient, the system (100) comprising:
one or more servers (101),
a medical imaging unit (108) coupled to the one or more servers (101), the one or more servers (101) comprising instructions, which when executed causes the one or more servers (101) to perform the method steps as claimed in any one of claims 1 to 13.
